Europäisches Patentamt

(19) European Patent Office

Office européen des brevets

(11) EP 0 782 860 A1

(12) EUROPEAN PATENT APPLICATION
published in accordance with Art. 158(3) EPC

(43) Date of publication:
09.07.1997 Bulletin 1997/28

(51) Int. Cl.⁶: A61K 39/385, A61K 39/00,
C07K 14/00, C07K 19/00

(21) Application number: 96922046.6

(22) Date of filing: 10.07.1996

(86) International application number:
PCT/ES96/00145

(87) International publication number:
WO 97/02838 (30.01.1997 Gazette 1997/06)

(84) Designated Contracting States:
AT BE CH DE DK ES FI FR GB GR IE IT LI LU MC
NL PT SE

(30) Priority: 12.07.1995 ES 9401953

(71) Applicant: Martin Oncina, Francisco Javier
10600 Plasencia (ES)

(72) Inventor: Martin Oncina, Francisco Javier
10600 Plasencia (ES)

(74) Representative: Elzaburu Marquez, Alberto et al
Elzaburu S.A.,
Miguel Angel, 21
28010 Madrid (ES)

(54) POLYMERIZED VACCINES

(57) Process for obtaining polymerized vaccines consisting in the polymerization between themselves, of antigenic proteins produced in a pathological process; the proteins to be polymerized have a molecular weight lower than 60 kilo-daltons (from 60 to 70 kilo-daltons) since they induce lymphocytic responses of the TH2 type (immunodepressors). The polymerization may be carried out by gradual synthesis, protein to protein, following the fundamentals of conventional processes for the synthesis of peptides, or by polycondensation (the election method) by means of alkylating agents, in restrictive concentration, with two activated groups capable of joining together two proteins by binding their two amino groups. The resulting polymers are selected (molecular weight between 100 and 200 kilo-daltons) by the column fractionation process with molecular sieves; the resulting selected polymers induce an antigenic response similar to the proteins forming them, but under control of the lymphocytes TH1 (immonunopotentiators).

EP 0 782 860 A1

## Description

This invention consists of a new design and process for preparing vaccines, based on the polymerization of peptides less than 60 kilo-daltons, originating from the different microorganisms against which the vaccination is sought, for specifically obtaining homo-polymers and/or hetero-polymers, of those referred antigenic peptides, with a final molecular weight greater than 70 kilo-daltons, it being desirable, although not critical, not to exceed 300 kilo-daltons by molecular weight; this polymer thus obtained, administered to the animal subject of the vaccination, will provoke a TH1 or cellular-type immunological response against those peptides, polymerized starting from the initial ones produced by the different germs. The optimal size of the polymer, for an ideal response, ranges between 100 and 150 kilo-daltons. This invention refers, likewise specifically, to obtaining these polymers by employing the classical methods (Curtius, Fischer, Bergmann, Zervas, Merrifield and Denkewalter, and their modifications) and/or polycondensation by polymerizing agents, based on the chemistry of the carbon, lineal or cyclical, that have the minimum presence of two alkylating functional groups on amino groups (carbonyl, acid, acid halogen, anhydride, azide, alkyl esters, isocyanate, dialkyl-phosphorous-amines, halogen on two different carbons or the combinations of these functional groups), which in some mild pH (alkaline or neutral) conditions, depending on the pK of each protein to be polymerized, join the functional (primary or secondary) groups of the different aminoacids which form the peptide or protein to be polymerized.

A characteristic of this patent, in using the classical techniques and their modifications for peptide synthesis, is the direct use of these, instead of aminoacids, to synthetize end proteins larger than 70 kilo-daltons.

Another characteristic of this patent is the use of polymerizing agents, comprised by a chain of short carbons (from 2 to 8 C) or cycle of carbons (from 4 to 6 C), with two active functional groups, preferably equal, which can join the amino groups of the proteins to form a larger protein; the polymerization is produced in restrictive conditions of the polymerizing agent, whose amount is deduced by a mathematical formula depending on the mass of the starting protein and the mass of the desired end polymer.

Lastly, another characteristic of the invention, against the polymers with carrier proteins, consists of polymerizing the greatest possible number of antigenic proteins, originated in a pathological process, and less than 60-70 kilo-daltons, to induce, due to their final size, a suitable response to the CD4-TH1 memory lymphocytes and specific for these antigens and, owing to the abundance and diversity of epitotes thus presented in the polymer, to avoid possible presentation restrictions due to the larger histocompatibility complex.

Basically the invention can be defined as an original biochemical application to a new biological concept.

State of the Art relative to vaccines:

Since the beginning of medicine it was known that individuals who recovered from the first attack of an infectious disease developed resistance against it, which led to the first experiments, in 1713, with the smallpox vaccine.

A vaccine is a preparation which, administered to an individual, induces specific resistance, from the immunological viewpoint, but not necessarily complete, against an infectious disease.

A good vaccine should be safe, effective, long lasting, stable and economic.

Clasically three basic types of vaccine agents have been employed:

- Toxoids:
  Diphtheria and tetanus owe their serious action to the bacterial endotoxines, the infection being trivial at the invasion site, such that protection against the disease is by immunizing with toxoids, which are acellular preparations of toxines treated with formaldehyde, adsorbed in aluminium salts as adjuvant.

- Inactivated vaccines:
  The majority of the bacterial vaccines (whooping cough, cholera, typhoid, etc.) employ dead microorganisms, except BCG, and have the drawback, in general, of needing large and multiple doses.

- Live and attenuated vaccines:
  By comparison with bacterial vaccines, viral vaccines usually, in their majority, utilize live agents weakened by going through animal cell lines (monkey, chicken embryion, etc.), having as drawback the possible inclusion of viable adventitious agents, possible relapse into virulence, physical instability and counter-indication in pregnancy and immunosuppression.

The new trends in vaccines allow their classification into two categories:

- Replicants:
  This vaccine production system employs live pathogene micro-organism strains, attenuated in bacterias by removal or induction of mutations, non-reversible, in codifier genes of key enzymes in the metabolic paths essential for the normal growth of micro-organisms; in the case of the viruses, which are obligatory endocellular parasites, the preceding process described is not, generally speaking, suitable and thus some more complex ADN viruses (herpes, poxvirus, etc.) codify their own enzymes from the ADN metabolism, such as timilidate-cyclase, so that the inactivation of this enzyme has an attenuating effect on the virulence, but the potential of this modification for vaccine purposes has still not been explored; in the Sabin polio vaccine, only ten changes in the nucleotides distinguish it from its virulent procreator.

Another method is the use of viral vectors, by introducing, through genetic engineering, genes which codify the suitable proteins inside the bacterial vaccine strains (for example, food poisoning) or virus ones, the most researched system being insertional mutation of the genome of the virus of the vaccine.

Moss, Paoletti and collaborators were the first to describe a method by which extraneous genes could be incorporated, by recombination "in vivo", inside the viral ADN. Many proteins have been expressed in this form and the method has become a research tool, as well as a potential way of developing vaccines, the immunogenic gen of the rabia glycoprotein, which has been incorporated to the virus of the vaccine, constituting an example of specific application. Recently a live transmission system has been developed, by the production of chimeric poliovirus, which has facilitated the production of virus mutants in which the antigenic loops have been replaced by foreign protein sequences and which, therefore, express the antigenic properties of these proteins (foreign), as well as the unmodified epitotes of the polioviruses,

- Non-replicants:

The expression of immunogenic proteins, through genetic engineering, has been possible with the advent of gene cloning and the expression techniques in bacterias, yeasts, mammal cells and insect cells.

The synthetic peptide vaccines consist of small peptide sequences which induce antibodies which, in turn, recognize the protein from which those derive. Anderer showed, in 1963, that a peptide of the protein of the virus envolute of the tobacco mosaic, joined to a proteic carrier, induced antibodies which neutralized the virus. The application of this technology to the development of new vaccines advanced, in the decade of the 1970's, with the advent of cloning and sequencing of the nucleic acids which made it possible to predict the aminoacid sequences of the relevant proteins. The chemical synthesis of peptides in solid support, conceived by Merrifield, is the technique used for obtaining the Patarroyo vaccine against malaria, consisting of the synthesis of small peptides, from 15 to 20 aminoacids, which join to a carrier protein; this method has the drawback, given the size of the real antigen, of the possibility of restriction due to the larger histocompatibility complex of the vaccinated individual and, moreover, the localization of the antigenic sites and the tertiary structure of the fused peptide with respect to the original protein.

The anti-idiotypical vaccines are based on the complementary nature of the interaction between an antigen and the combination site of the antibody which recognizes this antigen. The site of joining the antigen (idiotope) of an antibody (Ab1) is, physics-chemically, a mirror image of the epitope of the antigen recognized by that antibody.

In consequence, the antibodies (Ab2) which recognize the idiotype of Ab1 imitate the original antigen and can, therefore, act as substitute antigens capable of inducing antibodies (Ab3) of specificness similar to Ab1 for recognizing the original antigen.

The inducing of antibodies by anti-idiotypical vaccines has been demonstrated in various systems, but the responses are low and depend, often and critically, on the dose of anti-idiotypical antibody, so that their therapeutical impact is, for the time being, slight.

The presentation of the antigen also has importance in the immune response, and thus the need for the adjuvants, whose mechanism is complex, amongst which the complete Freund adjuvant stands out, with important side effects, or aluminium hydroxide gel, much utilized in human vaccines to enhance the synthetic vaccines response.

The polymeric presentation, by incorporating the antigenic proteins or peptides in larger structures, usually increases their immunogenicity, as occurring with the fusion or assembling of the protein of the core of the hepatitis B virus with a peptide sequence of the glosopede virus, but the increase of immunity is greater against the global protein than against the specific sequence. The viral glycoproteins have better responses when incorporated in liposomes or forming micellular complexes with Quil A glycoside, or when incorporated into slow release systems such as the copolymers of lactic acids and glycol or soluble crystal.

The analysis, up to this point, of the state of the art is based on the data contributed by Battle J.L.. and Murphy F.L. in Vaccine Biotechnology (London: Academic Press, 1989) and by Brostoff J., Scadding G.K., Male D. and Roitt I.M. in Clinical Immunology (London: Gower Medical Publishing, 1991).

As an example of the current trends, in the most original work for the design of new vaccines, we outline the following experiences:

- Tam and collaborators (J. Exp. Med., Vol. 171, January 1990, 299-306) have developed a vaccine against malaria, consisting of a nucleus of seven lysines with eight dendrite arms, from 10 to twenty aminoacids, with a high superficial density of antigenic peptides and a molecular weight that exceeds 10,000 daltons.

- Wang and collaborators (Science, Vol. 254, October 1991) have designed a synthetic octameric peptide uniting eight sequences of the third variable region (V3) of the 120 glucoprotein of HIV-1, on a hepta-lysil nucleous which bears eight amino-terminal reagent groups serving as carrier for eight V3 peptides, latched by synthesis in solid phase. This octameric peptide seems to produce, in guineapigs, a persistent and potent antibody response by comparison against the conjugates with bovine albumin.

The contrbutions by Tam and Wang have, as disadvantage, the blocking of acid groups on the lysine.

- Hart and collaborators (J. Immunol., 145, 1990, 2677-2685 and Proc. Natl. Acad. Sci. USA, Vol 88, November 991, 9448-9452) synthetized, using tercbutoxycarbonyl chemistry on solid support, hybrid peptides starting from an epitote (T1), N terminal of the gp-120 of the VIH involute, of the T cells with epitotes, of the B cells, hydrophylic, of the V3 region of the gp-120 of VIH (SP10 sequences). This T1-SP10 hybrid induces, in goats and primates, T-helper anti-VIH cell responses, against the T1 region of the gp-120 and high neutralizing anti-VIH antibody titles, against the SP10 region of V3 of gp-120 of the VIH. They subsequently designed diverse peptides to enhance the cellular cytotoxicity fusing T1 and SP10 with sequences, of 12 aminoacids (519-530), of the fusion domain of the gp-41 and others of peptides of the MN and III-B strains of the VIH.

- Stanhope and collaborators (J.I.D., 68, July 1993, 92-99) have tested the inducement of cytolitical CD4 responses, of the human T lymphocites, by a recombinant vaccine of gp-160 of VIH-1, which consists of a completely glycosilated form of gp-160, isolated starting from infected cells by coexpression of the T7 bacteriophage, purified and formulated with an adjuvant containing deoxycolate, aluminium hydroxide and thimerosal.

Respecting the vaccines against polysaccharides and lipopolysaccharides, the tendency is to utilize carrier proteins, as has been done for the following bacterias:

- Streptococos of the B group (Madoff et al, J. Clin. Invest. 94(1), July 1994, 286-92 and Marques et al. Infect. Immunol., 62(5), May 1994, 1593-9).

- Haemophylus influenzae (Meyer et al., Monatsschr. Kinderheilkd., 141 (10), October 1993, 770-6).

- Pneumococo type 9V (Lu et al. Infect. Immunol., 62(7), July 1994, 2754-60).

Other systems utilized employ formaldehyde, which is polymerized, or polyethylenglycol, which due to the presence, in both, of -0H groups, set the proteins joining to their carboxyl groups, forming complexes of greater molecular weight; the use of N-polyvinylpyrrolidone exercises its action setting the amino groups on the =C=0 groups of the pyrrolic ring joined to the polyvinyl by the N, whereby macromolecules are formed by union of the proteins to the carbonyl group, in form depending on possible steric impediments, forming multiple Schiff bases. A disadvantage of these systems, which increases the immunity, is the formation of networks or clusters, whereby an optimal presentation of the diverse antigen determinants is hampered.

At the present time, despite the continuous development of new vaccination systems, many challenges remain to be solved, VIH, due to its great social impact, being the most relevant one, as well as malaria, on being extremely widespread and in view of the modest results with the Patarroyo vaccine.

Criticism of the state of the art relative to the vaccines:

Few safe and effective vaccines exist in relation to the number of pathogen agents against which immunization would be desirable.

The classical attenuated live vaccines and, to a lesser degree, the modern replicants are unstable, the danger of reverting to virulence unable to be ruled out and not usually recommendable in pregnancy and immunesuppression conditions.

Vaccines, dead or inactivated, the classic and non-replicant modern ones, suffer from not, in many of them, producing a persistent immunising response, which provokes the need for successive enhancer and/or reminder doses, and, in others of them, the immunising response, exalted in a specific aspect (example: cytotoxicity with gp-160 of VIH), is not sufficient to check the action of the infectious agent; in other cases, such as in the Patarroyo vaccine against malaria, there is a modest percentage of success, probably by restriction of the greater histocompatibility complex, on being a small peptide with a single epitote (15-20 aminoacids).

Another specific aspect, of the non-replicant vaccines, is the insertion of one or a few antigen proteins, or their strands, in another protein which acts as carrier to strengthen the immunity, but if we perform a critical analysis, reviewing the literature on the immune responses against the vaccines of this type, we shall gather that, basically, two types of response exist, depending on the type of carrier protein and manner of setting:

A.- If the antigns are set in multiple fashion on a nucleous constituted by a small peptide, with end result of a protein not exceeding 65,000 daltons, they produce a predominantly humoral response under control of the TH2 lymphocyte subpopulation.

If the union of the antigen to the peptide nucleous is carried out by means of the carboxylic groups of the former, as in the case of the polylysine nucleii, the response is less than if the union is performed by means of the amino groups of the antigen to the peptide nucleous, as occurs were this constituted by polyglutamite.

B.- If the antigens join to a protein of acknowledged immunological effectiveness (larger than 65 kilo-daltons), the result is, obviously, an end protein larger than 65,000 daltons, producing a good immunity reponse, under control of the TH1 lymphocyte subpopulation, against the globality of the fusion protein, the specificness of the response being, however, proportional to the amount of antigen fused, in relation to the total fusion protein and, from the point of view of the infection, the effectiveness is only against this particular fused protein from amongst all those produced by the germ, which are variable throughout their biological cycle.

State of the art relative to the synthesis of proteins:

Fischer designed, in 1903, a process for the synthesis of peptides, based on the use of the acid chlorides of different aminoacids (or proteins) derivatives as acylating medium. Since the acid chloride groups would be partly destroyed in aqueous mediums, it is better to carry out the reaction in indifferent solvents, adding a second molecule of the amino component to collect the C1H formed. Ways of avoiding this difficulty consist of adding pyridine as second molecule with N to collect the C1H or the use of carbobenzoxy groups (M. Bergmann and L. Zervas in 1932).

Th. Curtius proposed, in 1904, the synthesis of peptides with activated carboxyl groups, which can be prepared starting from acid hydracides, in the ordinary way by nitrous acid. It is necessary to protect, by acylation, the free amine groups which are labile against the nitrous acid, while the NH groups of the peptide bonds, of slower reaction time, and the acylated amine groups are not attacked in the azide formation conditions. In the real synthesis, the nitrohydride acid is free, which decomposes immediately with release of elemental nitrogen.

Another acylation possibility is by the mixed anhydrides of carboxylic acids with inorganic acids which have the advantage that only the organic acyl group is lost to the combination it is sought to acylate.

The isocyanate group allows activation of the amine group so that it acts as one of its derivatives which is so slightly basic that it cannot accept the proton of the carboxyl group and possesses reaction capacity sufficient to transfer the nitrogen rest to this carboxylic group. St. Goldschmidt and M. Wick (1952), starting from studies by H. Staudinger (1922), were the first to use this system of transformation of the amine into isocyanate rest.

Another process for synthezizing peptides is based on acylation, with the protected amine groups, by the use of ester groups with alkyl rests with strong capacity for acting, activating positively, as with the esters of nitrile, of p-nitrophenol and thioesters (R. Schwyzer 1955).

The synthesis in solid phase, designed by Merrifield in 1962, consists of a system in which the initial portion of the chain remains anchored to a solid support by a covalent bond, whereby the synthesis can be carried out by successive additions without needing intermediate purifications. The formation of the peptide bond demands the activitation or either the carboxylic group or of the amine group (dicylce-hexyl-carbo-diimide is used as coupling agent). Protector agents are used (e.g. : tercbutyloxycarbonyl on the NH group) on the groups whose reaction is not desired. At the end the protein is released from the setting polymer with BrH or FH.

In the polycondensation, used by Denkewalter, the system of the azide is of interest, in alkaline and nitrous medium, at pH 7.4 and 37°C.

More up to date processes for peptide synthesis employ hydridizing of the the genes of the proteins by means of genetic engineering techniques.

Description of the Immunology basics:

It is an accepted theory worldwide that crossing over, by action of the specific antigen, of the immunoglobulines of the membrane of the B lymphocytes activates the tyrosine-cynases which lead the cell to the G1 phase (Celada & colls., Inmunolog□a B◁sica, Barcelona: Pub. Labor Universitaria, 1994, p. 384), but this theory cannot be sustained if it is taken into account that the lysozyme of egg white (Amit A.G. et al., Science, 233, 1986, p. 749), protein with an approximate mass of 14 kilo-daltons, induces the production of three different antibody clones, whose strands Fab, confronted simultaneously with the lysozyme, unite at a different site, as gathered from the structural analysis with X-rays, occupying an irregular area of 700 scare angstr`ns each so that, on the three simultaneously joining the lysozyme, they occupy the totality of the surface of the antigen, it not being possible for the lysozyme (with three different epitotes) to have crossed over with the surface immunoglobulines of the B lynphocite, which are monoclonal, to activate the signals which lead to the production of antibodies against it.

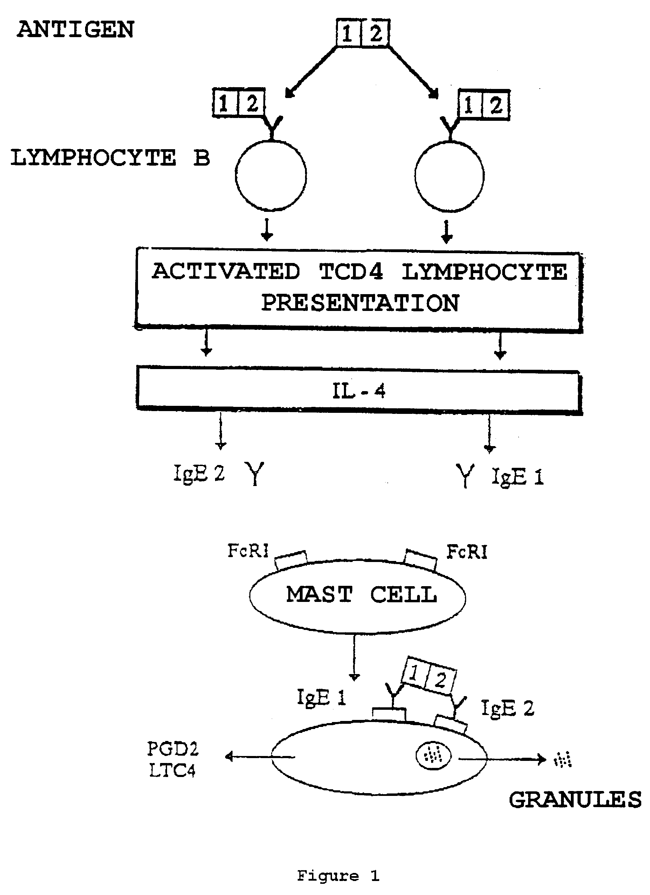

The crossing over of the surface immunoglobulines, by the antigens, can only be carried out on the IgE set in the surface of the mast cells (see Figure 1) and by the polysaccharides and lipo-polysaccharides (by reiteration of the antigen chemical sequence) in the surface of the B lymphocytes.

The foregoing has led to a critical review of the immunity system (review by the author with 247 biographical references analyzed) where, inter alia, the following conclusions are established:

1.- In the study of the immune-reactivity, the variability of the blastogenic response leads, on occasion, to different results which raises the need for multivaried experiments and statistical analyses to improve the conclusions. Amongst the possible causes of error, with contradictory results in the work of different researchers, free manuronic and glucuronic acid can be considered, which are powerful stimulators of the lymphocytes and macrophages, produced during the electro-phoresis of badly purified commercial alginates, and the presence of fetuine (gp-40), contained in the foetal serum of cattle, which is employed in the majority of cell cultures in diverse ratios, being cell growth activator at 10% (probable TH2 stimulation) and the cells remaining quiescent at 0.5%).

2.- The production of the different immunoglobulines and interleucines is governed by the TH1/TH2 relationship, of the T-CD4 lynphocite subpopulations.

3.- The membrane antigens (CD), of the cells presenting antigens, are different in the B lymphocytes and in monocyte/macrophage lineage and like cells.

4.- The Th1 subpopulation is produced by interaction, through specific antigns in the respective membranes of the T-CD4 lymphocytes pre-activated with the monocytes/macrophages and like cells.

5.- The TH2 subpopulation is produced by interaction, by specific antigens in the respective membranes of the T-CD4 lymphocytes pre-activated with the B lymphocytes.

6.- The response by the immunity system is against an antigen, so the origin of the TH1/TH2 relation has to be looked for in the physics-chemical characteristics of the protein or set of proteins, not recognized as their own, which trigger their reaction.

7.- The size, the dose and entry path, influence the interactions between the antigen and the immunity system which, along with the circumstances of the host (genetic determinism, nutritional state, age, presence of infections, pharmacological treatments, etc.), produce variations in the immunity responses by some individuals compared with others.

8.- The number of epitopes of an antigen protein is proportional to the size of the same so that, in the same individual, the ratio between the different antibody clones varies with time, those which react best being selected.

9.- The immunoglobuline and interleucine patterns, produced by the domination of the TH2 lymphocyte subpopulation, occurs, in leser or larger degree, in diverse pathological processes, the T-CD4 lymphocyte occupying a central role in their regulation, meaning that the analysis is fundamental, of the conditions which provoke their differentiation in the TH1 or TH2 subpopulation, as well as their different ratios.

10.- The most powerful immunogens are proteins with a molecular weight between 70 and 50 kilo-daltons, the role of the protease inhibitors being known (lately against the VIH protease) in the improement of the immunity response.

As conclusion, it is gathered that the size of the antigen is crtical in the type of immunity response triggered, and the number of epitotes present in the polymer, constructed with one or several proteins of the agent, is inversely proportional to the possibility of restriction by the larger histocompatibility complex of the individual vaccinated.

The current vaccines do not take into account the classification of the antigen depending on its molecular weight, or the influence which, for that reason, derives in the type of immunity response.

The advantage of this model of vaccine consists in its being a polymer, of one or several antigens, related with the pathological process, so that the diversity, abundance and repetition of the antigen determinants leads to a more powerful and diverse immunological response, moreover resulting in a cheap vaccine, non-replicant, easy to prepare, TH1 type immunogen and of low restriction due to the greater histocompatibility complex.

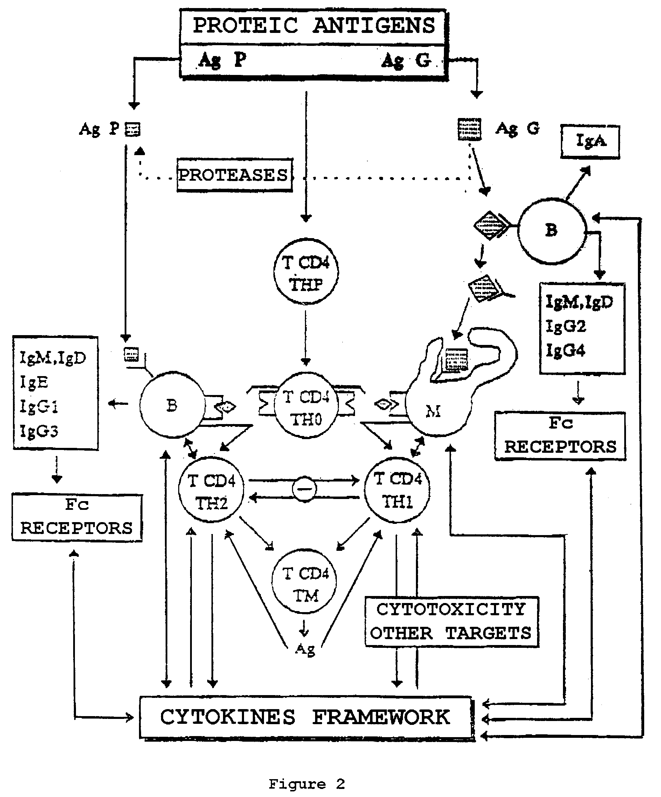

The invention is based on the original idea that the production, in a pathological process, of deregulated (extraneous) proteins less than 60,000 daltons, is responsible for the immunity discontrol that leads, in a series of diseases, to immunedepression, due to dominance of the CD4-TH2 lymphocyte subpopulation (see scheme in Figure 2).

The study of the antigens involved in the diverse phases of those processes in which immunodepression is produced, under dominance of a TH2 pattern, such as certain viral processes (VIH, HTLV, EBV, hepatitis, influenza, etc.), bacteial (tuberculosis, leprosy, borreliosis, etc.), parasitic (malaria, leishmaniasis, etc.), oncologic (oncoproteins ras, crkl, etc.), allergic (plants, fungi, ticks, latex, etc.), etc., show the correlation between the size of the dominant antigens, the response triggered and the response mechanism proposed, as theoretical ground for this new model of vaccines; there is, moreover, the interesting coincidence in the majority of the proccesses with run with greter or lesser degree of TH2 responses, that the most representative antigens have a size close to 24 kilo-daltons, which is the size of the Fab strand of the immunoglobulines, and the prevalence of these antigens is related with the inadequate immunity response, giving greater force to the model suggested, which alows the following relation to be defined:

$$\text{Pattern TH1 / TH2} = f\,(A\,/\,B + 2C) * T$$

f:    function.
A:    number of molecules larger than 70 kilo-daltons.
B:    number of molecules larger than 70 kilo-daltons.
C:    number of molecules larger than 24 kilo-daltons.
T:    Antigen exposure time.

Description of the physics-chemical basics:

The invention attempts to produce, specifically, proteins larger than 70,000 daltons, preferably between 100,000 and 150,000 daltons, starting from proteins or peptides smaller than 65,000 daltons, by polymerization amongst them, by polymerization of these with proteins or peptides larger than 65,000 daltons and by specific union (peptide with peptide or protein with protein) of the peptides or proteins smaller than 65,000 daltons with peptides or proteins larger than 65,000 daltons.

It is not pertinent to describe the techniques of detection, separation, purification, characterization, quantification and determination of mass of the proteins to be polymerized, inasmuch as these are to be found amply described in numerous manuals.

It is assumed, to start with, that the proteins are to be found lyophylized, weighed and the mass known (average if mixture of various proteins).

- Prior considerations repecting the proteins:

The potential diversity of the proteins to be polymerized can force variations in the pH range (basically neutral and alkaline) and in the temperature (depending on the denaturalization).

Protein solvents, as biological macromolecules, have a conduct determined by three fundamental factors:

- They are very large
- They can interact strongly amongst one another or with the solvent
- They frequently have electrical charges.

Proteins, as macromolecules, can basically adopt the following formations:

- Statistical chain (random coil):
  This is a lineal polymer having low resistance to the rotation around the bonds of the chain and in which there are few interactions of the lateral groups, so that it tends to adopt a sort of free formation (likewise occurring in the polysaccharides and lipopolysaccharides).

- Bar type macromolecule:
  The proteins of this type normally have a simple secondary helicoidal structure. The properties of their solvents depend greatly on the length of the bar, the diameter being of lesser importance.

- Globular macromolecule:

In these proteins there are strong interactions amongst the lateral chains and they tend to group in dense globular formations similar to squat sphere (oblate) or elipsoids (prolate).

Formation of proteins:

A native protein is a molecule of regular structure, very organized, while in a denaturalized protein a folding and untangling is produced, which involves an increase in entropy. On the denaturalization being produced, interactions can arise between the solvent and the protein which produce variations of entropy, which add to or subtract from the denaturalization entropy variation. Whenever a substance is heated its entropy (disorder) increases. A denaturalized protein can adopt more formations than the native one, which has influence on the equilibrium. The stability of those of the real formations of the proteins is a complex phenomenon and the effect of the solvent cannot be underestimated, since the solution-solvent interactions can play a very important role.

Protein solvents:

Protein solvents have a non-ideal conduct, inasmuch as they interact strongly with one another or with the molecules of the solvent.

An ideal solvent adjusts to the entropy (ideal) of the law of mixtures, in that the size of the molecules of the solution are of the same order as that of the molecules of the solvent, such that solute and solvent can freely interchange their positions in an hypothetical structure, but in the protein solvents, that are not ideal, such does not occur due to the absolute size of the particles; each protein is many times larger than a solvent moleule, in fact, an aminoaid of the protein has a size nearer that of the molecule of the solvent, resulting as if the molecules of the solute (in aminoacid units) had to move together in group.

The distribution of the molecules of solute, in a proteins solvent, does not follow the laws of chance, since the centre of each protein cannot be situated in a certain volume that is determined by the volumes the rest of the molecules occupy.

Equilibrium of charges:

A large and complex protein can interact with small molecules at different sites, independent or cooperative, and each site of union has its association and disassociation constant interrelated; if the sites are different (carboxyl, amine, etc.), the analysis of the cooperativeness is difficult and the values of K are very different, since the ionic force of the solvent also complicates the analysis, on the electrostatic effects of the bond being another form of cooperativeness.

The protein-proton equilibrium (e.g., ribonuclease, PM 14 kilo-daltons) is studied depending on the disassociation constants. Not all the potentially neutralizable groups of the ribonuclease (36 in all) are evaluated in the region of pH 2 to pH 12. It is to be expected that the four guanydil groups neutralize towards pH 12, but not all neutralize, and to continue above pH 12 is difficult inasmuch as irreversible changes can occur in the protein.

The evaluation curve, as expected, contains several intervals, since the carboxyl groups usually have pK values comprised between 3 and 4, the histidine and the alpha-amino groups neutralize towards pH 7, the tyrosine and $NH_3^+$ groups of the lateral chains have pK values close to 10 and the arginines neutralize to pH 12.

Additionally, information can be obtained by a spectrophotometry evaluation of the tyrosines, due to the ionized tyrosine spectrum being very different from the non-ionized one, the ionization of these isolated groups being measurable by absorbance to 295nm (they can be ionized by groups to different pH).

The ionic force has to be taken into account, inasmuch as, to the extent this increases, the electrostatic effect on the ionization of the protein diminishes. The presence of protons can be strongly tied up with the formation change, so change in the size of the protein during the evaluation has to be avoided.

These prior considerations are going to allow a better selection of a strict pH and temperature range for those proteins whose polymerization offers a low yield in mild conditions (pH 7.4 and 25□C), but it is necessary, once the polymerization is performed, to bring the protein (polymerized) to these mild conditions prior to the separation by means of molecular screens.

- Prior considerations respecting the polymerization:
  The -C00- groups do not show any acylating actiity and the $NH_3^+$ groups cannot be acylated as a result of the basic properties of the same, so it is only possible to form a peptide bond between them at high temperatures, which is inviable in the case that interests us; accordingly, it is necessary to start from carboxyl or amino groups richer in energy which do not form hybrid ions.

To achieve the foregoing, it is possible to proceed two ways:

A.- Activating the carboxyl group and transforming it into one of its derivatives permitting acylation of the amino group:

Example:

$$-NH-CHR-CO-O-SO_3H + H_2N-CHR'-CO- ---> ------> -NH-CHR-CO-NH-CHR'-CO-$$

- Acid chlorides (Fischer), which have the drawback of being destroyed in aqueous alkaline mediums, whih forces the reaction to be carried out in indifferent components with one molecule (pyridine) to collect the C1H group.
Example:

$$-HN-CHR-CO-Cl + H_2N-CHR'-CO- ---> -HN-CHR-CO-NH-CHR'-CO-$$

- Azides of carboxylic groups (Curtius), which have similar properties to the alkyl halogens and do not attack the peptide or acylated N, those of lower groups being explosive.
Example:

$$R-CO-N_3 + H_2N-CHR'-CO- ---> R-CO-NH-CHR'-CO- + 3/2N_2 + 1/2H_3$$

- Isocyanate, very unstable in water, formed starting from acylazides on losing N by mild heating in inert solvents.
Example:

$$-HN-CHR-COOH + O=C=N-CHR'-CO- ---> --------> -HN-CHR-CO-NH-CHR'-CO-$$

- Alcohyl ester groups:
R. Schwyzer, 1955, achieved increase in the activity of the components to be acylated with their protected amine groups, by the use of ester groups with alkyl rests, with strong capacity for activating positively, using nitrile esters of glycolic acid and of p-nitrophenol, and also thioesters and thiocarbonic acid.
Example:

$$Cbo-NH-CHR-CO-O-CH_2-CN + H_2N-CHR'-CO- ---> ---> Cbo-NH-CHR-CO-HN-CHR'-CO- + CH_2OH-CN$$

(Note: Cbo is carbobenzoxy and R is alkyl group).

B.- Activating the amino group to have it act as one of its derivatives being so little basic that it cannot accept the proton of the carboxyl group that has to form the amide group but which, on the other hand, possesss sufficient reaction capacity to transfer the nitrogen rest to this carboxyl group.

- Transformation of the amine group in isocyanate rest, which can combine with the free carboxylic acids (H. Staudiger, 1922), with intermediate formation of anhydride of the carbamide acid, with removal of $O_2$ to give the acid amide. St. Goldschmidt and M. Wick applied alpha-isocyanate on carbobenzoxylated aminoacids for the synthesis of peptides.
Example:

$$Cbo-NH-CHR-COOH + O=C=N-CHR'-CO- ---> --> Cbo-NH-CHR-CO-NH-CHR'-CO-$$

- Formation of P-N bond contained in the amides of the di-alkylphosphorous acid, which is sufficiently active to react with the carboxyl groups with formation of acid amides (G.N. Anderson, 1952).
Example:

$$H_2N-CHR'-CO- + (RO)_2POH ---> (RO)_2P-NH-CHR'-CO- (RO)_2P-NH-CHR'-CO- + -CHR''-COOH ---> CHR''-CO-NH-CHR'-CO-$$

- The phosphorous-azo-combinations of the amine group raise the problem of the final presence of polymers with phosphorous, difficult to quantify and to remove the proteic polymer.

The possibility of formation of dicetopiperacines is difficult, in the case of proceeding directly with proteins, owing to the theoretical separation of the amine and functional carboxyl groups, such that it is not of great importance to protect the groups of the proteins that intervene in the reaction and, therefore, we are not going to find ourselves with the difficulty of removing the protector groups of the amine groups; notwithstanding, sometimes it may be necessary to protect groups that it is not of interest that they might undergo acylation (-COOH, NH2, -OH, -SH, etc.) and which are not found at the end of the molecule that form their head or tail so that, in the specialized literature, numerous methods are available, developed in the thirties, for protection and release of the protector group, which fall outside this patent.

As examples of the diversity of protector groups, we refer, for the amine group, to carbetoxylation (Fischer), arbobenzoxylation (Bergmann and Zervas), carbobenzoxylation with benycl group substituted by p-bromobencyl or p-nitrobencyl, bencylthiourethanes, trifluoacetyl, phtalyl rest, tritile, p-toluensulfonyl, bencyl rest directly linked to the N of the amine (without interposition of urethane), etc., or indirect protection by derivates of alpha-halogenated acid, alpha-azide derivatives of fatty acid, carboxylic alpha-ketoacid derivatives, etc., and, for the protection of the acid group, the formation of salts that neither act as acylation agents nor lose protons.

Brief description of the drawings:

Figure 1 shows the formation of two different IgE clones, against an antigen, and the crossing over of the Ige, in the surface of the mast cell, by union of the IgE to the two antigenic determinants, which cannot occur in the B lymphocyte.

Figure 2 explains the process followed by a small antigen (P), less than 70 kilo-daltons, which is endocyted by the B lymphocyte and presented to the T lymphocyte, inducing a TH2 phenotype and the process followed by a large antigen (G), larger than 70 kilo-daltons, which cannot be endocyted by the B lymphocyte (restriction due to the size of the receptor), but attaches to the membrane immunoglobulines of the B lymphocytes which are specific for some of their determinants, whereby it can be fagocyted by the macrophage and presented to the T lymphocytes, inducing a Th1 phenotype.

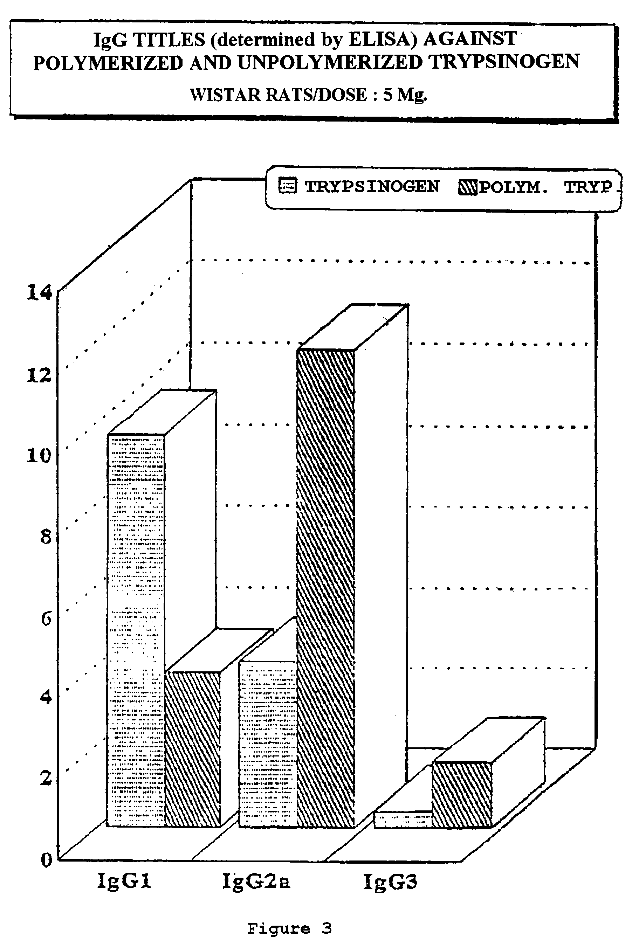

Figure 3 shows the change in the antibody titles (determined by ELISA) against the unpolymerized and polymerized (oblique lines) bovine trypsinogene (horizontal lines), 20 days after administration of 5 mg. Of each one to Wistar rats.

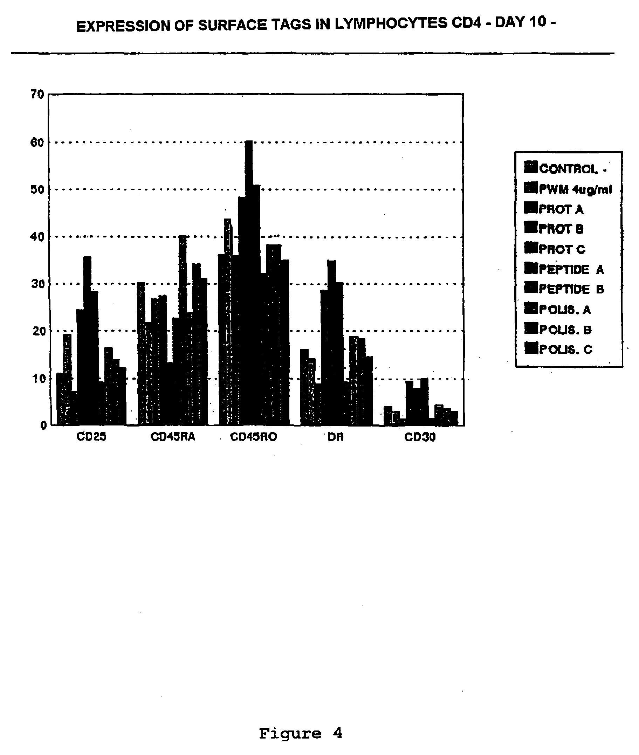

Figure 4 shows the marking levels of the surface of T-CD4 human lymphocytes after 10 days of culture with trypsinogene (prot A), polymerized trypsinogene (prot B), potato beta-amylase (prot C, 200 kDa), peptide B (SIGMA P-4409, 40 kDa) and peptide A (peptide polymer B).

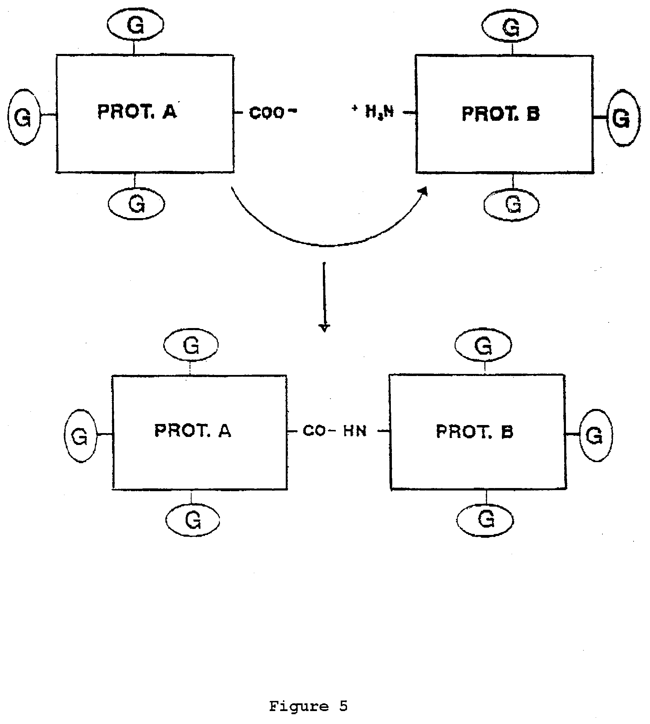

Figure 5 shows a scheme of the gradual peptides synthesis, in which the carboxyl-amino (amide) union is carried out with the rest of the protected functional groups (G) ; the selective deprotection of functional groups allows the polycondensation with glutaraldehyde, in restrictivee concentration, in which the bond of the carbonyl groups and amino is carried out with the rest of the protected functional groups (G), two protein molecules joining with one glutaraldehyde one, or more, in a subsequent step, depending on the final desired calculated molecular weight.

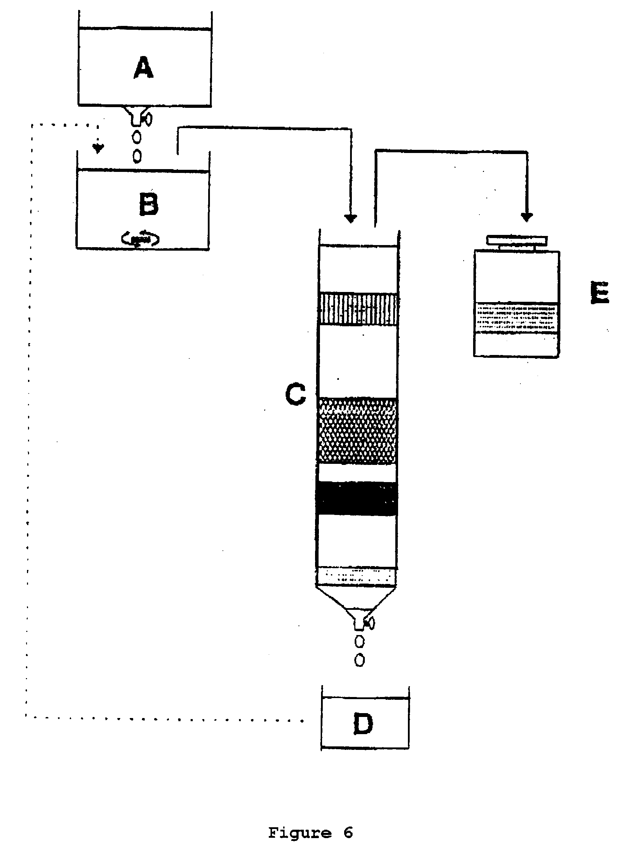

Figure 6 is a scheme of the process of polymerization bypolycondensation, where in container A the polymerizing agent(glutaraldehyde) is placed and in B the protein or mixture of proteins to be polymerized. The separation of the suitable polymers is carried out in column C (molecular strainers:Sephadex G-75, G-100, G-150 or G-200, depending on the masses tobe selected), the filtrates within the range selected (containerD) constitute the polymer obtained for the vaccine (E); the filtrates of lesser range than that selected can be recycled.

Detailed description of the invention: For polymerization of the antigenic proteins the following methods can be used: A.- Gradual synthesis protein by protein: Consists of joining two peptides, one by one, using the known classical methods.

- Solid phase:

Use of solid support in columns, as in the Merrifield system(synthesis in solid phase), but directly using the proteins to be polymerized, instead of aminoacids, with protection of the free carboxyl groups of the proteins attached to the resin and protection of the amino groups with terc-butoxycarbonyl chloride(Boc system), before treating the acid group of the protein with dicycle-hexyl-carbodiimide (DCC) and forming the activated 0-acyl-urea which is going to react with the amino groups,unprotected, of the protein attached to the resin (ghloromethyl groups of the polystyrene) by an acid group. Examples of possibilities of end polymers with starting proteins A, B and C:

A + A = AA; AA + B = AAB; AAB + C = AABC, etc.

- Liquid phase:

If the resin is foregone and attachment of the acid group of the column replaced by acid protector groups, the Merrifield condensation can be made in liquid phase, carrying out the following stages:

1.- Container with n mols of protein A with its acid groups protected.

2.- Container with n mols of protein B with its amino groups protected (Boc) and activated acid groups (DCC).

3.- Discharge, drop by drop and with shaking, of the container with the activated protein B on the container with protein A, at a weak alkaline pH.

4.- Release and separation of the protector groups.

5.- Separation of the polymers by means of columns with moleculars strainers.

Examples of possibilities of end polymers with starting proteins A and B:

If A and B are equal:

$$A + A = AA$$

If A and B are different:

$$A + B = AB + ?AA + AB + BB\ ?$$

This same gradual synthesis process in liquid phase can be carried out, in a similar way, in aqueous medium or inert medium, according to the procedure employed and depending on the stability of the protein allowing activation of the desired group, using proteins with the acid groups activated and the other groups protected and discharging them on the proteins (equal or different) through which amino groups it is sought topolymerize, or utilizing proteins with the amino group activated and the rest protected and discharging them on the proteins (equal or different) through which acid groups it is sought to polymerize, it even being possible to perform this synthesis, were there to be a low yield, with a group of proteins with the amino group activated and the other group of proteins with the acid group activated.

This gradual synthesis process in liquid or solid phase allows the formation of polymers with their components sufficiently orderly and of controlled size, although from the practical perspective of obtaining vaccines, the formation of a certain percentage of polymers differing from those foreseen is of no importance.

Once the polymerization process has finalized, selection is proceeded with of the suitable polymers for filtration with molecular strainers, at pH 7.4 and less than 36□C.

B. Polycondensation.

Polycondensation is the quickest, cheapest and simplest system for the formation of polymers.

There is the additional advantage, if the polymerizing agent acts on the amino groups, that maintenance is allowed of the functional acid groups being the ones responsible, basically, for the antigenic action and, what is more, on being a process in which the proteins do not always unite by the same functional groups, they avoid the overlapping of epitopes by a fixed type of union, whereby, practically, all remain exposed and the possible restriction due to the larger histocompatibility complex is avoided.

The polycondensation has always been made in excess of polymerizing agent, which provokes the formation of reticular clusters which, although able to increase the immunogenicity provoking TH1 responses, provoke a loss in the presentation of the suitable antigenic surface of the protein of origin and exhaust the presence of all the amino groups present in the protein, which can have consequences for the immunity response, due to loss of epitopes, owing to the restriction in the presentation due to the larger histocompatibility complex.

The polycondensation system, here patented, is based on the use of the polymerizing agent, which has two active groups, being carried out restrictively on the basis of the ratio of one mol of polymerizing agent per two mols of protein to be polymerized, which ration can vary depending on the size of the starting protein and the desired final size, on the basis of the following equation :

$$G = P \times M \times q / 2N^2$$

G :   Grams of polymerizant per volume of solvent.
P :   Grams of protein per volume of solvent.
M :   Molecular weight of the polymerizing agent.
Q :   Final molecular weight desired for the polymer (Q > 2N)
N :   Molecular weight of the protein to be polymerized.

(Note : the volumes of the solutions of polymerizer and protein should be equal, the factor, non-critical, for both volumes being the achievement of a protein solvent no greater than 10% (recommendable) and, depending on the volume necessary for that percentage of protein in solvent, the necessary grams are calculated of polymerizant for an identical volume, whereby the equation can be simplified in both parts).

The polymerization can be carried out on the amino groups, activated or not, of the protein to be polymerized, which join two by two, through the action of diverse agents, which are compounds pertaining to the chemistry of carbon, such as :

- Molecules with two carbonyl groups :
Give rise to Schiff bases.

For the reactions indicated, R is a chain of from 2 to 8 carbon atoms, depending on the functional groups and the solubility, the presence not being critical of other radicals in the carbons that do not support the functional groups : R can also be a cycle of 4 to 6 carbon atoms, although this option is less interesting, given the physiological consequences of molecules of this type.

He polymerization systems recommendable, because of the type of solvents and the lesser occurrence of undesirable or toxic compounds, are the agents with two carbonyl groups, two mixed anhydride groups and two alkyl esters.

Example of the resulting polymers :

A + B + C + POLIMERIZANTE --> A-B-C + A-C-B + B-C-A + B-A-C + A-A-B + A-A-C + B-B-A + B-B-C ...

Example 1.- Polymerization with glutaraldehyde :

Polymerization is proceeded with of 25o mg. Trypsinogene of bovine pancreas (Sigma Chemical), with a molecular weight of 24 kilo-daltons, which, in Wistar rats, induces an increase of IgE and IgG1 and, therefore, TH2 type lymphocyte responses.

To calculate the grams of glutaraldehyde necessary for obtaining an end polymer of 250 kilo-daltons, we apply the formula, described on page 26, $G = P \times M \times Q / 2N^{2,}$ from which it is gathered that 5.4 mg. Of glutaraldehyde are necessary to obtain the desired polymers.

To facilitate handling, we dilute the 250 mg. Of trypsinogen in 100 ml. (or 25 ml.) of bidistilled water and the equivalent of 5.4 mg. Of glutaraldehyde (of a grade 1 :50% aqueous solution) in another final volume of 100 ml. (or 25 ml.).

- Molecules with two aklyl esters :
Example :

$$NC-CH_2-O-OC-R-CO-O-CH_2-CN + H2N-R' ---> ----------> R'-NH-CO-R-CO-NH-R'$$

- Molecules with two different halogen atoms in C :
Due to the halogen they need inert solvents.
Example (with chlorine) :

$$ClCH_2-R-CH_2Cl + H_2N-R' ---> R'-NH-CH_2-R-CH_2-NH-R'$$

- Halogens of halogen-acids :
These require inert solvents due to the presence of halogen.
Example (with chlorine) :

$$ClCH_2-R-COCl + H_2N-R' ---> R'-NH-CH_2-R-CO-NH-R'$$

It is also possible to activate the protein forming azides, isocyanates or dialkyl-phosphorous-amines, as convenient, on the amino or acid groups, which can give the following reactions :

- Protein isocyanate more diacid:
Very reactive in water, needing inert solvents.
Example :

$$(Protein)-N=C=0 + H00C-R-C00H -----> -----> (Protein) -NH-C0-R-C0-NH (Protein)$$

- Protein azide more diacid :
Very reactive, toxic, complex reactions,

- Dialkyl phosphorous amines more diacid.
Example :

$$HOOC\text{-}R\text{-}COOH + (R'O)_2P\text{-}NH\text{-}R'' \dashrightarrow R''\text{-}NH\text{-}CO\text{-}R\text{-}CO\text{-}NH\text{-}R''$$

Example :

$$(H)R'\text{-}CO\text{-}R\text{-}CO\text{-}R'(H) + H_2N\text{-}R'' \dashrightarrow \dashrightarrow R''\text{-}N\text{=}CR'(H)\text{-}R\text{-}CR'(H)\text{=}N\text{-}R''$$

- Molecules with two mixed anhydride groups :
  Example :

$$HO_3S\text{-}O\text{-}OC\text{-}R\text{-}CO\text{-}O\text{-}SO_3H + H_2N\text{-}R' \dashrightarrow R'\text{-}NH\text{-}OC\text{-}R\text{-}CO\text{-}NH\text{-}R'$$

- Molecules with two acid halogens :
  These are very reactive with water which implies the need for inert solvents.
  Example (chloride) :

$$Cl\text{-}OC\text{-}R\text{-}CO\text{-}Cl + H_2N\text{-}R' \dashrightarrow R'\text{-}NH\text{-}OC\text{-}R\text{-}CO\text{-}NH\text{-}R'$$

- Molecules with two azide groups :
  The azides of lower series are explosive, having conduct similar to the halogenated derivatives (inert solvents) and giving complex reactions.
  Example :

$$N_3\text{-}OC\text{-}R\text{-}CO\text{-}N_3 + H_2N\text{-}R' \dashrightarrow R'\text{-}NH\text{-}CO\text{-}R\text{-}CO\text{-}NH\text{-}R$$

- Molecules with two isocyanate groups :
  These are unstable in water, very reactive, irritant and toxico, giving complex reactions, so not usually utilized.

The container containing the trypsinogene is buffered, prior to achieving the final volume, with a saline phosphate buffer (SPB), for a pH of 7.4 (the pH should be maintained through the process).

The containers are arranged as described in Figure 6, and the glutaraldehyde is mixed, drop by drop, on the protein, at a temperature of 15-20°C and with shaking.

Once the mixture is completed, this is maintained, with intermittent shaking, during 16 hours, before proceeding to the separation, it being unnecessary to paralyze the mixture with glycine-0H.

The separation is carried out, amongst other possibilities, in columns with Sephadex G-200/Sepharosa 4B (Pharmacia), and the fraction filtered and selected, composed by the polymers of 250 kilo-daltons and mixtures of larger sizes, being dialyzed and concentrated by evaporation under vacuum until 10 ml.

Subsequently, the 10 ml. Solution, containing the polymer, can be lyophylized.

Administration to Wistar rats, following filtration with filters of 0.2. micras (Millipore), of 5 mg. Of the polymer dissolved in 1 ml. Of bidistilled water, to which aluminium hydroxide has been added, induces an increase of IgG2a and IgG4 and a drop of IgG1 (Figure 3) and a reduction in the rate of IgE, measured by the passive cutaneous anaphylactic activity test (Mora I., Wong D., Life Sci. 1969 ;8 ;813), as well as an increase in human lympocyte cultures (see Figure 4) of the membrane antigens CD25, CD45RO, HLA-DR and CD30 (Activated lymphocyte response of Th1 memory type).

Example 2.- Polymerization of proteins :

It is possible to proceed, as in Example 1, to polymerize proteins less than 70 kilo-daltons produced, in the pathological process, by an infectious agent, for example VIH.

The VIS proteins to be polymerized, as example, are p12, p17, p24, p31, p34, p41 and p51.

The amount of glutaraldehyde necessary is calculated depending on the percentages of mass of each one with respect to the total and their respective molecular weighs.

The steps are performed as in Example 1, giving as result different polymers of approximate molecular weight, basically, at 250 kilo-daltons and a mixture of ppolymers with higher molecular weight.

Examples of possible results :

p17-p24-p31-p34-p41-p51-p17-p24-p31
p24-p31-p34-p41-p51-p17-p24-p31-p17
p31-p34-p41-p51-p17-p24-p31-p17-p24

p51-p24-p24-p51- ....... etc.

The possibility of likely poly-peptides or proteins depends on the number of potential combinations between the different starting peptides.

These poly-peptides induce an increase of the lymphocytes T CD4, CD45R0, CD30, CD25, HLA-DR which control the immunity measured by cells, increase the production of antibodies of high affinity (IgG2a and IgG2b), diminish the rate of IL-4 and increase the rate of IFN-gamma activating the macrophages.

The restriction controlled by the larger histocompatibility complex in the suitable immunizing response is avoided, in the process here described, by the variety of starting peptides utilized and the random form of union between them, which allows the potential presentation of all the epitopes for the selection of those genetically suitable.

## Claims

1. A process for obtaining polymerized vaccines, obtained by means of the polymerization, by gradual synthesis or polycondensation, of peptides or proteins less than 70 (+/- 15%) kilo-daltons, utilizing polymerizing agents in restrictive concentration, whose number of mols is equilibrated depending on the amount and physics-chemical characteristics of the proteins to be polymerized, giving as result an end protein larger than 70 kilo-daltons, which is a polymer of the starting proteins and induces a TH1 type immunity response against those peptides or antigenic-pathological protein, and consisting of the following processes :

   a) Activation of the carboxyl groups of the proteins, with protection of the functional groups rest, and adding on another protein or the same, with the amino group activated, or not, slowly and at neutral or alkaline pH : or activation of the amino groups of a protein, with protection of the functional groups rest and adding on another protein or the same, with the carboxyl group activated or not, slowly and at neutral or alkaline pH ; or utilization of an alkylant agent, in restrictive concentration or not, with two active functional groups for adding, slowly, on one or several proteins, in a neutral or alkaline pH solution, and giving, as in the other two processes above, a polymer superior to 70 kilo-daltons, whose antigenicity is, basically, the sum of its component proteins but under control of the TH1 lymphocyte subpopulation induced by its greater molecular weight.

   b) Fractioned filtration, by membrane filters, of the resultant polymers in point b) and making them suitable for administration, in the appropriate doses, as vaccines.

2. The processes described in Claim 1, in which the participant antigenic proteins, activated or not, are less than 70 kilo-daltons and pertain to microorganismos of the Mycobacterium tuberculosis groups (p10, p20, etc.), M. leprae (p12, p14, p15, p28, p35, p31, p20, p40, p30, p31, p45-60, etc.), Plasmodium genus (p17, p45, p27, etc.), Leishmania genus (p14-18, p32, etc.), Schistosoma genus (p28 x 2, etc.), Treponema genus (p15, p42-47, etc.), Borrelia genus (p22, p39, etc.), Cándida genus (p36, p44, etc.), VIH-1 virus (p6, p9, p12, p13, p14, p15, p17, p18, p19, p20, p24, p26, p31, p34, p36, p41, p51, p55, p63, p68, etc.), VIH-2 virus (proteíns similar to VIH-1, p11, p64, p34, p15, p26, p55, p36, etc.), HTLV I y II virus proteíns similar in size to VIH-1 y 2), hepatitis B virus (p14, p17-18, p21, p30, p46, p20, etc.), hepatitis C virus (p24-35, p9, etc.), hepatitis delta virus (p18, p21, p29, p34, etc.), Borna virus (p24, p35, p38, etc.), influenza virus (p14.4, etc.), prions (p30, etc.), crkl oncoproteín (p39), v-sis oncoprotein (p28), c-sis oncoprotein (p24), kip-1 oncoprotein (p27), c-mos oncoproteín (p39), ras oncoproteín (p21), v-src oncoproteín (p60), ATP tumoral marker (p22), PSA tumoral marker (p34), alergenes such as G. paniculata (p20, p29 y p39), P. praetense (p21-30), L. perenne (p11, p12, p17, p27 y p56), H. anuus (p24, p32 y p55), A. maritima (p5-38), A. fumigatus (p24, p34 y p38), A. alternata (p25- 60), C. herbarum (p13 y p25), D. pteronissimus (p24-25 y p18), D. farinae (p17), cod (p13, p25, p37 and p63), egg (p35 y p40-45), bee poison (p16 and p18), latex (p21 and p 14-75), bovine hair (p20), bird feathers (p20-30), dog hair (p18 and p25), horse hair (p27 to p67), etc.

3. The processes indicated in Claim 1, in which the polymerization methods by polycondensation, restrictive or not, there being of application, on the selected proteins, polymerizing-alkylant, lineal molecules of 2 to 8 carbon atoms or cyclical of 4 to 6 carbon atoms, with two functional groups, equal or different, such as the molecules with two carbonyl groups (dialdehydes of the oxalic, malonic, succinic, glutaric, adipic, azelaic, suberic diacids and their derivatives, such as cyclobutanodione, cyclopentanodione and cyclohexandodione), of two mixed anhydride groups (of the diacides referred to in the preceding parentheses and the sulfuric and phosphoric acids), diacide chlorides (of the diacids referred to in the first parentheses of this claim), diacide azides (of the diacids referred to in the first parentheses of this claim), diacide isocyanates (of the diacids referred to in the first parentheses of this claim), alkyl esters (of the diacides referred to in the first parentheses of this claim), derivatives halogenated with two halogens (F, Cl, Br, I) in two different C of lineal molecules of 2 to 8 carbon atoms, halogens of omega-halogenacids (F, Cl, Br, I) of the acetic, propionic, butanoic, pentanoic, hexanoic, heptanoic and octanoic acids, as well as utilization of the diacides, referred to in the first parentheses of this claim), used as polymerizants agaisnt proteins activated in their amino groups by the formation of isocyanate, azide and dialkyl-phosphorous-amine groups.

4.  The process described in Claim 1, in which tthe polymerizing agent is glutaraldehyde in restrictive concentration suitable to the number of mols of proteins or peptides to be polymerized as described in Example 1 of the detailed description of the invention.

5.  The polymerization process referred to in Claim 1, in which the polymerization agents utilized, referred to in Claim 3, form reticular clusters with the proteins referred to in Claim 2.

6.  The process referred to in Claim 1, with the agents referred to in Claim 3, in which the proteins, referred to in Claim 2, activate at a neutral or alkaline pH.

7.  The process referred to in Claim 1, in which, besides polymerizing the protein, the resulting polymer is isolated, by molecular screens.

8.  The products, homo-polymers or hetero-polymers, obtained by use of the process described in Claim 1 and the agents listed in Claims 3 and 4, with the proteins indicated in Claim 2, that have application as vaccines.

9.  Claim 8, in which the proteins are polymerized with polysaccharides or lipo-polysaccharides produced by the same infectious agent.

10. Claims 8 and 9, referring to the application for antibody production.

11. Claims 8, 9 and 10, referring to the process of Claim 1, with the agents described in Claims 3 and 4, in their application to proteins of pathological processes for provoking TH1 type responses.

12. The industrial and commercial application of the content of the preceding claims.

Figure 1

Figure 2

IgG TITLES (determined by ELISA) AGAINST POLYMERIZED AND UNPOLYMERIZED TRYPSINOGEN

WISTAR RATS/DOSE : 5 Mg.

Figure 3

**EXPRESSION OF SURFACE TAGS IN LYMPHOCYTES CD4 - DAY 10 -**

Figure 4

Figure 5

Figure 6

## INTERNATIONAL SEARCH REPORT

| International application No. |
|---|
| PCT/ES 96/00145 |

**A. CLASSIFICATION OF SUBJECT MATTER**

$Int.Cl^6$: A61K 39/385 A61K 39/00 C07K 14/00 C07K 19/00

According to International Patent Classification (IPC) or to both national classification and IPC

**B. FIELDS SEARCHED**

Minimum documentation searched (classification system followed by classification symbols)

$Int.Cl^6$: A61K

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 4180562 A (MCINTIRE FLOYD C ET AL) 25 December 1979 (25.12.79) the whole document | 1-8,10,12 |
| X | EP 0367306 A (CBF CORP BIOLOG FARMA SA) 9 May 1990 (09.05.90) the whole document | 1-8,10,12 |
| X | FR 2270891 A (AGENCE NATIONALE DE VALORISATION DE LA RECHERCHE (ANVAR)) 12 December 1975 (12.12.75) the whole document | 1-12 |
| X | GB 1164313 A (HOECHST A.) 17 September 1969 (17.09.69) Page 3, line 4 - line 50; examples 1-6; claims 1-11 | 8,10 |
| A | WO 9406472 A (PASTEUR INSTITUT; GENGOUX CHRISTINE (FR); LECLERC CLAUDE (FR)) 31 March 1994 (31.03.94) the whole document | 1-12 |
| A | EP 0117934 A (UNIV OHIO) 12 September 1984 (12.09.84) the whole document | 1-12 |

| ☒ | Further documents are listed in the continuation of Box C. | ☒ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 November 1996 (22.11.96) | 3 December 1996 (03.12.96) |

| Name and mailing address of the ISA/ S.P.T.O. | Authorized officer |
|---|---|
| Facsimile No. | Telephone No. |

Form PCT/ISA/210 (second sheet) (July 1992)

**INTERNATIONAL SEARCH REPORT**

International application No.

**PCT/ES 96/00145**

C (Continuation). DOCUMENTS CONSIDERED TO BE RELEVANT

| Category* | Citation of document, with indication, where appropriate, of the relevant passages | Relevant to claim No. |
|---|---|---|
| A | CHEMICAL ABSTRACTS, Vol. 107, No.1, 1987<br>Columbus, Ohio, US;<br>abstract No.5413,<br>LECLERC,C. ET AL: "A synthetic vaccine constructed by copolymerization of B and T cell determinants"<br>page 509; column 2;<br>abstract<br>& Eur.J.Immunol. 1987, 17(2)269-273 | 1-12 |
| A | BIOLOGICAL ABSTRACTS, vol.96, n°.5, 1993<br>Philadelphia, PA, US;<br>abstract n°.52015,<br>YANG XI, ET AL: "Chemically modified antigen preferentially elicits induction of Thl-like cytokine synthesis patterns in vivo"<br>abstracts<br>& J.Exp.Med. 178 (1):349-353, 1993 | 1-12 |

Form PCT/ISA/210 (continuation of second sheet) (July 1992)